# EUROPÄISCHE PATENTSCHRIFT

(11) **EP 1 300 108 B1**
(45) Veröffentlichungstag und Bekanntmachung des Hinweises auf die Patenterteilung: **28.02.2007**
(21) Anmeldenummer: 01890279.1
(22) Anmeldetag: 01.10.2001
(51) Int. Cl.: A61B 3/113

(54) **Verfahren zur Erfassung, Auswertung und Analyse von Blicksequenzen**
Method for obtaining, evaluating and analyzing sequences of vision
Méthode permettant d'obtenir, d'évaluer et d'analyser les sequences de vision

(43) Veröffentlichungstag der Anmeldung: 09.04.2003
(73) Patentinhaber: Pfleger, Ernst, Univ. Prof. Dipl.-Ing. Dr., 1010 Wien (AT)
(72) Erfinder: Pfleger, Ernst, Univ. Prof. Dipl.-Ing. Dr., 1010 Wien (AT)
(74) Vertreter: Gibler, Ferdinand

(56) Entgegenhaltungen:
- EP-A- 0 125 808
- EP-A- 0 240 336
- GB-A- 2 212 354
- US-A- 5 555 895

## Beschreibung

Die Erfindung betrifft ein Verfahren zur Erfassung, Auswertung und Analyse von Blicksequenzen einer Testperson mittels eines Blickerfassungssystems,
- wobei das Blickfeld der Testperson durch eine nach vorne gerichtete und starr mit dem Kopf der Testperson verbundene erste Kamera erfaßt und in einem Blickfeldvideo aufgezeichnet wird,
- die Bewegung der Pupille der Testperson durch eine zweite Kamera, die ebenfalls starr mit dem Kopf der Testperson verbunden ist, erfaßt und in einem Augenvideo aufgezeichnet wird
- und das Augenvideo und das Blickfeldvideo auf ein Videosystem aufgezeichnet und zeitlich synchronisiert werden,
- wobei für jedes Einzelbild des Augenvideos die Pupillenkoordinaten ermittelt werden,
- die Korrelationsfunktion zwischen Pupillenkoordinaten auf dem Augenvideo und den Koordinaten des entsprechenden Blickpunktes, d.h. des Punktes, den die Testperson fixiert, auf dem Blickfeldvideo ermittelt wird
- und nach erfolgter Ermittlung der Korrelationsfunktion für jedes Einzelbild aus den Pupillenkoordinaten auf dem Augenvideo die Koordinaten des entsprechenden Blickpunktes auf dem Blickfeldvideo extrapoliert werden.

Derartige Verfahren sind im Stand der Technik bekannt. Bei diesen werden die Pupillenkoordinaten aber entweder gar nicht oder nur sehr ungenau und auf sehr aufwendige Weise erfaßt.

Aus der US 5 555 895 A ist ein Verfahren und eine Vorrichtung zur Analyse der Augenbewegung eines Patienten bekannt.

Die EP 125 808 A2 zeigt ein Verfahren zum Beobachten der Augenbewegung.

Aus der GB 2 212 354 ist ein Apparat für das Detektieren der Schrichtung eines Auges bekannt.

Die EP 240 336 A2 offenbart ein Verfahren zur Generierung einer Beschreibung der Verteilung der Zuschauzeit von Leuten, die kommerzielles Fernsehen schauen.

Die Erfindung hat zur Aufgabe, die bekannten Systeme zu verbessern und ein Verfahren der eingangs genannten Art vorzustellen, bei dem eine zuverlässige Erkennung der Pupillenkoordinaten für jedes Einzelbild des Augenvideos mit möglichst geringem technischem Aufwand erfolgen kann.

Erfindungsgemäß wird dies dadurch erreicht, daß zur Ermittlung der Pupillenkoordinaten für jedes Einzelbild des Augenvideos mit einem Bilderkennungsprogramm automatisch
- die Kontraste der Pupille zum Umfeld registriert werden,
- alle Punkte des Einzelbilds, die dunkler als ein eingestellter Dunkelheitsgrad sind, gesucht werden,
- mit diesen Punkten eine der Pupille entsprechende Dunkelfläche voll erfaßt und abgegrenzt wird, und
- der dem Pupillenmittelpunkt mit den Pupillenkoordinaten entsprechende Schwerpunkt der Dunkelfläche ermittelt wird.

Durch dieses Verfahren wird zunächst die gesamte Pupille als Dunkelfläche erfaßt. Die Registrierung des Kontrastes zwischen der dunklen Pupille und der beinahe weißen Umgebung der Pupille ermöglicht eine besonders einfache automatische Erkennung der Dunkelfläche. Die Ermittlung des Schwerpunktes dieser Dunkelfläche erfolgt auf besonders einfache Weise und verlangt nur einen geringen rechnerischen Aufwand, wobei der Schwerpunkt aber mit großer Genauigkeit angegeben werden kann. Durch Gleichsetzung des Pupillenmittelpunktes mit dem Schwerpunkt der Dunkelfläche kann somit der Pupillenmittelpunktes auf besonders einfache Weise mit großer Genauigkeit ermittelt werden. Die Erfindung basiert auf dem im Stand der Technik bekannten allgemeinen Prinzip der Zusammenführung eines Augenvideos und eines Blickfeldvideos, und stellt durch die neu entwickelten optischen und mathematischen Verfahren im Hintergrund und durch die sich daraus ergebenden neuen Analyseverfahren eine wesentliche Innovation dar.

Um Fehler durch unvorhergesehene Störungen oder Lichteinfälle zu korrigieren, kann gemäß einer weiteren Ausgestaltung der Erfindung vorgesehen sein, daß nach der automatischen Ermittlung der Pupillenkoordinaten eine händische Korrektur der Pupillenkoordinaten erfolgt.

In Weiterbildung der Erfindung kann vorgesehen sein, daß zur Ermittlung der Korrelationsfunktion
- zunächst eine oder mehrere Musterblicksequenzen der Testperson auf einen oder mehrere bestimmte vorgegebene Passpunkte aufgenommen werden
- und die Zuordnung der Pupillenkoordinaten auf dem Augenvideo zu den Koordinaten des Passpunktes auf dem Blickfeldvideo ermittelt wird,
- indem für jedes Einzelbild im Augenvideo die Pupillenkoordinaten im Augenvideo ermittelt werden,
- die Koordinaten des Passpunktes im entsprechenden Einzelbild auf dem Blickfeldvideo ermittelt werden,
- die Pupillenkoordinaten im Einzelbild des Augenvideo den Koordinaten des Passpunktes im entsprechenden Einzelbild des Blickfeldvideo zugeordnet werden,
- dieser Datensatz abgespeichert wird,
- und aus allen Datensätzen, vorzugsweise durch quadratische Regression, die Pupillenkoordinaten auf dem Augenvideo und die Koordinaten des entsprechenden Blickpunktes auf dem Blickfeldvideo korreliert werden.

Durch dieses Verfahren erhält man eine Korrelationsfunktion, die auf den sich entsprechenden Koordinaten auf jedem Einzelbild des Augenvideos und des Blickfeldvideos basiert. Dadurch ergibt sich eine außergewöhnlich hohe Genauigkeit der Korrelationsfunktion, wobei die Ermittlung derselben auf einfache Art durchgeführt werden kann und zuverlässig ist.

Um eine weitgehend automatische Ermittlung der Korrelationsfunktion zu ermöglichen, kann in weiterer Ausbildung der Erfindung vorgesehen sein, daß die Koordinaten des Passpunktes auf jedem Einzelbild des Blickfeldvideos automatisch durch ein Bilderkennungsverfahren ermittelt werden.

Gemäß einer anderen Variante der Erfindung kann vorgesehen sein, daß die Koordinaten des Passpunktes auf jedem Einzelbild des Blickfeldvideos händisch mittels Mausklickverfahren ermittelt werden. Dadurch können diese auch bei schwierigen Bedingungen, wie z.B. bei nicht ausreichendem Kontrast oder unerwünschten Lichtreflexen durch Blitze etc., sicher ermittelt werden.

In weiterer Ausgestaltung der Erfindung kann vorgesehen sein, daß die Musterblicksequenzen durch Drehen des Kopfes der Testperson bei Fixierung eines einzelnen Passpunktes erhalten werden. Dies stellt eine besonders einfache Methode dar, unterschiedliche Positionen der Passpunkte auf dem Blickfeldvideo zu erhalten, wobei die Definition eines einzelnen physikalischen Passpunktes genügt.

Bei einer weiteren Ausführungsform der Erfindung kann vorgesehen sein, daß zwischen 25 und 100 Positionen des Passpunktes bzw. der Passpunkte verwendet werden. Dadurch ergibt sich die gewünschte sehr hohe Genauigkeit der Korrelationsfunktion bei gleichzeitig vertretbarem Rechenaufwand.

In weiterer Ausgestaltung der Erfindung kann vorgesehen sein, daß aus dem Blickfeldvideo und den ermittelten Koordinaten des Blickpunktes auf dem Blickfeldvideo ein Ergebnisvideo erzeugt wird, auf dem der Blickpunkt durch eine visuell gut sichtbare Markierung, insbesondere durch ein Kreuz, angezeigt wird. Dies ermöglicht eine besonders anschauliche und deswegen für eine schnellere Analyse der Blicksequenzen vorteilhafte Darstellung der Ergebnisse.

Gemäß einer weiteren Ausbildung der Erfindung kann vorgesehen sein, daß mehrere Kategorien definiert werden und der Blickpunkt für jedes Einzelbild einer Kategorie zugeordnet wird, indem für jedes Einzelbild überprüft wird, im Bereich welcher Kategorie der Blickpunkt liegt.

Dadurch ergibt sich die Möglichkeit, die große Fülle an Daten, welche sich durch die Auswertung der Blicksequenzen ergibt, übersichtlich nach Kategorien zu ordnen. Dadurch, daß nicht mehr die Pupillenkoordinaten jedes Einzelbildes, sondern nur noch die Koordinaten der unterschiedlichen definierten Kategorien weiterverarbeitet werden müssen, ergibt sich eine drastische Reduktion des Datenvolumens. Die Datenmengen können in komprimierter Form weiterverarbeitet und analysiert werden. Dabei kann der hoch technische Inhalt der Blickfeldanalyse besonders einfach aufbereitet und auch für den Laien ohne fachliche Qualifikation anschaulich dargestellt werden.

In weiterer Ausgestaltung der Erfindung kann vorgesehen sein, daß zur komprimierten Darstellung der zeitlichen Abfolge der Zugehörigkeit des Blickpunktes zu bestimmten Kategorien einer Blicksequenz ein Diagramm erstellt wird, welches zwei Achsen aufweist, wobei eine Achse einer Zeitachse entspricht und die andere Achse den einzelnen Kategorien entspricht, wobei für jedes Einzelbild ermittelt wird, in welcher Kategorie sich der Blickpunkt befindet und in dem Diagramm an der dem Zeitpunkt des Einzelbildes entsprechenden Höhe der Zeitachse und der der Kategorie entsprechenden Höhe der anderen Achse eine visuelle Marke eingetragen wird. Dadurch ergibt sich ein Diagramm, welches in übersichtlicher Weise die zeitliche Abfolge der Blickzuwendungen angibt und somit eine einfache und schnelle weitere Analyse ermöglicht.

Nach einer anderen Variante der Erfindung kann vorgesehen sein, daß zur komprimierten Darstellung der Verweildauer des Blickpunktes auf bestimmten Kategorien einer Blicksequenz auf ein Standbild
- für jede Kategorie ermittelt wird, bei welchen Einzelbildern der Blickpunkt in einer Kategorie liegt,
- die Verweildauer des Blickpunktes auf dieser Kategorie durch Addition der Dauer dieser Einzelbilder ermittelt wird, und
- ein Bild erstellt wird, welches dem Standbild entspricht, in dem im Bereich jeder Kategorie ein grafisches Element angeordnet ist, welches zumindest einen Parameter aufweist, der der Verweildauer proportional ist.

Damit ergibt sich ein Diagramm, welches in übersichtlicher Weise die Gesamtdauer der Blickzuwendungen zu einzelnen Kategorien angibt. Die wesentliche Information aus allen Einzelbildern des Blickfeldvideos und allen Pupillenkoordinaten der Einzelbilder des Augenvideos ist somit in diesem Diagramm zusammengefaßt, wobei lediglich ein einziges grafisches Element pro Kategorie verwendet werden muß.

Um ein Diagramm zu erhalten, welches in übersichtlicher Weise die Reihenfolge der Blickzuwendungen zu einzelnen Kategorien angibt, kann gemäß einer weiteren Ausführung der Erfindung vorgesehen sein, daß zur komprimierten Darstellung der Reihenfolge der Blickabfolgen eine perspektivische oder entzerrten Darstellungen der von der Testperson betrachteten Szene erstellt wird und in dieser jedem Blickpunkt ein Pfeil zugeordnet wird.

In Weiterführung der Erfindung kann vorgesehen sein, daß die Untersuchungssituation aufgezeichnet wird, und diese Aufzeichnung jeder Testperson vorgespielt wird, wobei deren Blicksequenzen mittels eines Blickerfassungssystems erfaßt und ausgewertet werden,
- wobei das Blickfeld der Testperson durch eine nach vorne gerichtete und starr mit dem Kopf der Testperson verbundene erste Kamera erfaßt und in einem Blickfeldvideo aufgezeichnet wird,
- für jedes Einzelbild des Blickfeldvideos die Koordinaten des entsprechenden Blickpunktes, d.h. des Punktes, den die Testperson fixiert, auf dem Blickfeldvideo ermittelt werden.

Dadurch kann das Blickverhalten unterschiedlicher Testpersonen sehr einfach für die selbe Untersuchungssituation analysiert werden. Dies ermöglicht es, das Blickverhalten abhängig beispielsweise vom Alter oder dem körperlichen Zustand der Testpersonen auszuwerten. Dabei können Untersuchungssituationen vorgegeben und reproduziert werden.

Die Erfindung wird unter Bezugnahme auf die beigeschlossenen Zeichnungen, in welchen besonders bevorzugte Ausfuhrungsbeispiele dargestellt sind, näher beschrieben. Dabei zeigt:
Fig. 1 eine schematische Darstellung des mit dem Kopf der Testperson verbundenen Teiles des Blickerfassungssystems;
Fig. 2 eine Darstellung eines eine Brille 3 umfassenden mit dem Kopf der Testperson verbundenen Teiles des Blickerfassungssystems;
Fig. 3 eine schematische Darstellung zweier Einzelbilder jeweils aus dem Augenvideo und aus dem Blickfeldvideo;
Fig. 4 eine Darstellung eines Videosystems zur Aufzeichnung der beiden Videosignale;
Fig. 5 die Darstellung einer Benutzeroberfläche zur erfindungsgemäßen Ermittlung der Pupillenkoordinaten (xa,ya);
Fig. 6 eine schematische Darstellung zweier Einzelbilder jeweils aus dem Augenvideo und aus dem Blickfeldvideo bei der Aufnahme einer Musterblicksequenz;
Fig. 7 die Darstellung einer Benutzeroberfläche zur erfindungsgemäßen Ermittlung der Korrelationsfunktion K;
Fig. 8 ein Einzelbild aus einem Ergebnisvideo;
Fig. 9 eine Skizze eines zu analysierenden Bildes;
Fig. 10 ein erfindungsgemäßes Diagramm zur komprimierten Darstellung der zeitlichen Abfolge der Zugehörigkeit des Blickpunktes B zu bestimmten Kategorien ki einer Blicksequenz;
Fig. 11 ein erfindungsgemäßes Diagramm zur komprimierten Darstellung der Verweildauer des Blickpunktes B auf bestimmten Kategorien ki einer Blicksequenz auf das in Fig. 9 skizzierte Standbild;

Die Aufgabe von Blickerfassungssystemen ist es, mit möglichst großer Genauigkeit darzustellen, auf welchen Punkt des Blickfeldes eine Testperson blickt, d.h. auf welchen genauen Punkt das Interesse bzw. die Aufmerksamkeit der Testperson gerichtet ist.
Dabei wird einerseits das Blickfeld durch eine nach vorne gerichtete und starr mit dem Kopf der Testperson verbundene erste Kamera 1 erfaßt. Weiters wird die Bewegung der Pupille der Testperson durch eine zweite Kamera 2, die ebenfalls starr mit dem Kopf verbunden ist, erfaßt. Starr verbunden bedeutet in diesem Zusammenhang, daß beide Kameras 1, 2 so mit dem Kopf der Testperson verbunden sind, daß sie sich mit diesem bewegen bzw. allen Bewegungen folgen, wobei aber die Bewegungsfreiheit des Kopfes und der Augen in keiner Weise eingeschränkt wird. Aus der Auswertung dieser beiden Aufnahmen ist es möglich, mit großer Genauigkeit anzugeben, auf welchen Punkt die Testperson gerade blickt. Es sind dadurch exakte Aussagen über Blickzuwendungen, Blickbindungen und Blickabsenzen möglich.
Solche Blickerfassungssysteme werden bevorzugt im Sicherheitsbericht, insbesondere im Bereich der Unfallforschung, aber auch im Bereich der Werbung oder bei anderen humanphysiologischen Untersuchungen eingesetzt.
Insgesamt stellt die Blickverhaltensforschung einen wesentlichen Baustein zur Erforschung von physiologischen Unfallursachen dar. Beispielsweise können durch umfassende Blickuntersuchungen neue Erkenntnisse für die Unfallaufklärung und Unfallrekonstruktion hinsichtlich menschlicher Leistungsgrenzen gefunden werden.
Üblicherweise werden z.B. besonders gefährliche Stellen im Straßenverkehr mit Blickerfassungssystemen untersucht. Eine mit einem solchen Blickerfassungssystem ausgestattete Testperson durchfährt dabei die gefährliche Stelle, wobei ihr Blickverhalten aufgezeichnet wird. Die Summe der dabei analysierten Blicke wird im folgenden mit Blicksequenz bezeichnet. Aus der Analyse des Blickverhaltens kann nachvollzogen werden, welche Wegweiser oder Signaltafeln aufgrund ihrer ungünstigen Plazierung nicht genügend Beachtung finden, oder wo besonders uneinsichtige bzw. wenig beachtete Stellen in einer Kreuzung liegen.
Im Bereich der Arbeitssicherheit, z.B. auf Baustellen kann untersucht werden, welche Gefahrenquellen erst sehr spät von der Testperson erfaßt werden, und welche Sicherheitsvorkehrungen hier notwendig wären.
Ein weiteres wichtiges Anwendungsgebiet von Blickerfassungssystemen liegt in der Analyse von Werbeplakaten oder Werbespots. Auch hier kann sehr genau erfaßt werden, welche Botschaften, Texte Logos etc. von der Testperson wie lange und in welcher Reihenfolge fixiert werden.

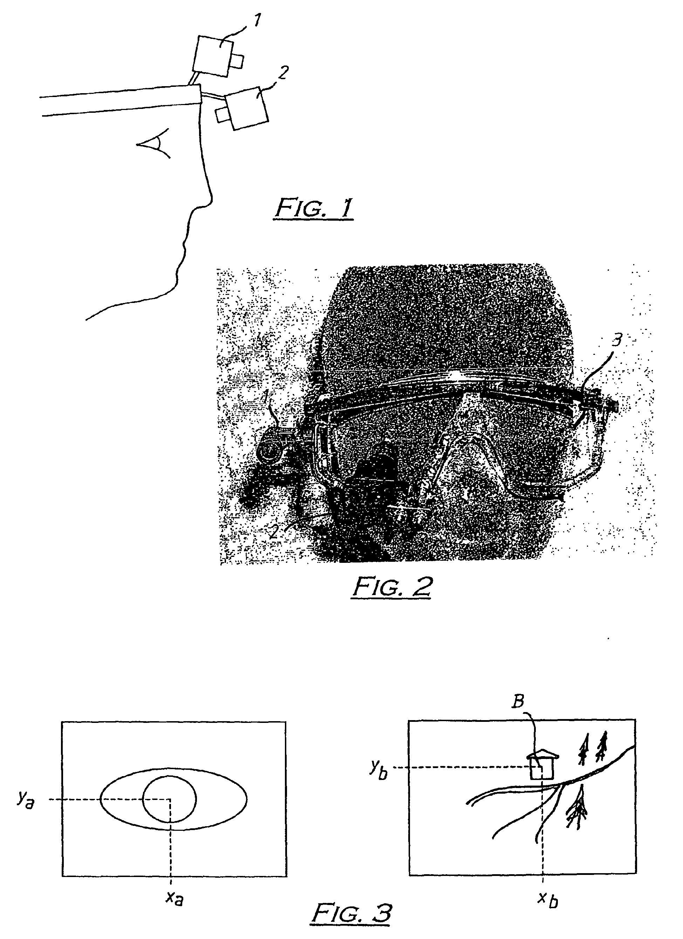

Fig. 1 zeigt ein Teil eines Blickerfassungssystems zur Durchführung des erfindungsgemäßen Verfahrens. Das Blickfeld der Testperson wird durch eine nach vorne gerichtete und starr mit dem Kopf der Testperson verbundene erste Kamera 1 erfaßt. Diese erste Kamera 1 gibt somit ein ungefähres Bild der Blickrichtung der Testperson, welche rein durch die Position des Kopfes definiert ist. Die erste Kamera 1 kann z.B. durch eine CCD-Farbkamera realisiert sein, die einen Großteil des Blickfeldes der Testperson aufzeichnet.
Vorzugsweise kann die erste Kamera 1 und/oder auch die zweite Kamera 2 zusätzlich mittels Software gesteuert werden und so den unterschiedlichsten äußeren Einsatzbedingungen angepaßt werden. Dies stellt sicher, daß durch die direkte Aufnahme der Pupille keinerlei Verzerrung des Pupillenbildes gegeben ist, bzw. durch die direkte Nähe zum Auge eine große Abbildung vorhanden ist und die Einrichtung insgesamt kleiner gehalten werden kann. Bisherige Verfahren stellen sowohl durch die Größe, als auch durch die generelle schlechtere Bildqualität erhebliche Ursachen für Unschärfen dar. Dies bedeutet nicht nur Erschwerungen im Gewicht des Blickerfassungssystems, sondern auch generelle Einschränkungen im Blickverhalten der Testperson, welche durch das erfindungsgemäße Verfahren vermieden werden. Daher kann das erfindungsgemäße Blickerfassungssystem auch von Probanden mit unterschiedlichen Kleidungen und Schutzmaßnahmen wie z.B. Helm ohne Einschränkungen verwendet werden. Es können daher auch sehr leicht unterschiedliche Kameras mit verschiedenen Objektiven je nach Versuchsanforderungen Anwendung finden.
Die qualitativ hochwertigen Kameras, die im erfindungsgemäßen System eingesetzt werden sind vorzugsweise mit einer Control Unit ausgestattet, die es ermöglicht automatischen Weißabgleich, Farbbalance und Belichtung vorzunehmen. Diese Werte sind üblicherweise auch manuell einstellbar. Durch diese Control Unit wird ermöglicht die Bildqualität den Versuchsbedingungen optimal anzupassen. Hiermit ist eine sehr hohe Bildqualität zur weiteren Analyse sichergestellt. Weiters besteht vorzugsweise die Option, den Bildausschnitt als digitalen Zoom elektronisch zu vergrößern. Andere Einstellmöglichkeiten haben in der Regel nur bedingten Einfluß auf das generierte Bild.

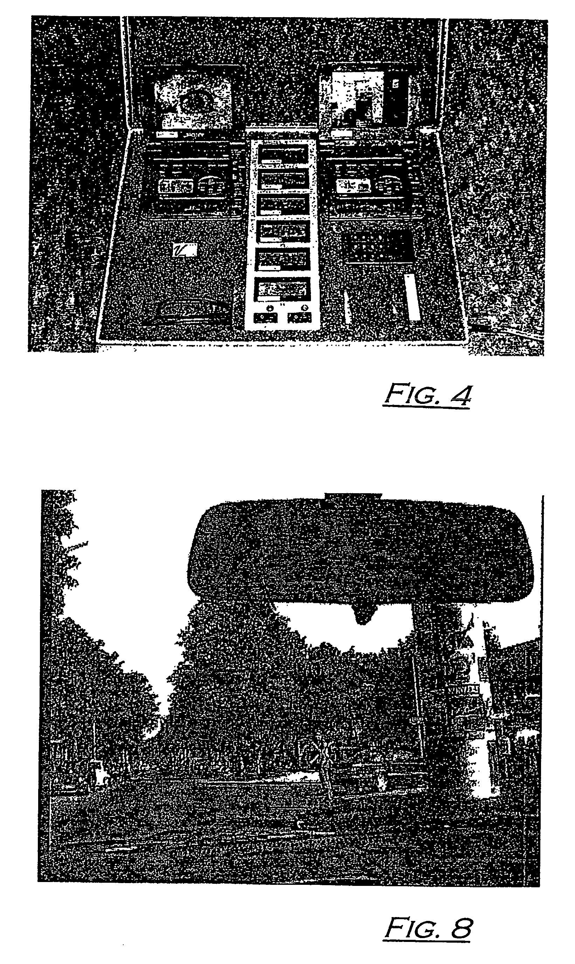

Die Bewegung der Pupille der Testperson wird durch eine zweite Kamera 2 erfaßt, die ebenfalls starr mit dem Kopf der Testperson verbunden ist, und auf eines der beiden Augen der Testperson gerichtet ist. Die zweite Kamera 2 kann beispielsweise durch eine s/w CCD-Kamera realisiert sein und die Augenbewegungen des rechten Auges registrieren. Die Erfassung der Pupillenposition durch die zweite Kamera 2 erfolgt bei den in den Figuren dargestellten Blickerfassungssystemen unmittelbar, wobei die zweite Kamera 2 direkt auf das Auge der Testperson gerichtet ist. Die Erfassung der Pupillenposition kann aber auch über optische Umlenksysteme wie Spiegel oder Glasfaserkabel erfolgen, mit denen das Bild vom Auge an die zweite Kamera 2 umgelenkt wird.
Die beiden Kameras 1, 2 sind beispielsweise auf einem Helm oder einer Brille 3 (vgl. Fig.2) oder einer ähnlichen vorzugsweise leicht auf- und abnehmbaren Trägereinrichtung aufgebracht, die starr mit dem Kopf der Testperson verbunden sind. Unter starr verbunden ist, wie oben bereite erläutert, zu verstehen, daß die Trägereinrichtung und beide Kameras 1, 2 allen Bewegungen des Kopfes folgen, wobei aber die Bewegungsfreiheit des Kopfes und der Augen in keiner Weise eingeschränkt wird. Die Anbringung der Kameras auf einer Brille 3 als leicht auf- und abnehmbare Trägereinrichtung ermöglicht eine besonders große Mobilität der Testperson und erlaubt eine sehr viel größere Versuchsvielfalt als bei herkömmlichen Systemen.
Natürlich ist es auch möglich, mehrere zweite Kameras 2 vorzusehen, beispielsweise um beide Pupillen der Testperson aufzunehmen. Auch können mehrere erste Kameras 1 vorgesehen werden, um das Blickfeld der Testperson vollständig zu erfassen, falls die Brennweite einer einzelnen ersten Kamera 1 hierfür nicht ausreichend ist.
Auf diese Weise können einzelne Blicksequenzen erfaßt und, wie im folgenden beschrieben, ausgewertet und analysiert werden. Die Bezeichnung Blicksequenz steht dabei für die Summe der jeweils aufgenommenen und analysierten Blicke.
Durch die beiden Kameras 1, 2 erhält man zwei, im folgenden mit Augenvideo und Blickfeldvideo bezeichnete und in Fig. 3 schematisch dargestellte Videosignale, welche auf ein in Fig. 4 dargestelltes Videosystem aufgezeichnet werden.
Die Bezeichnung Videosystem umfaßt hierbei alle Einrichtungen, die geeignet sind, Filmdaten aufzuzeichnen. Das Trägermaterial zur Aufzeichnung der Videosignale ist hier unwesentlich. Es können analoge Filmmaterialien wie auch Videobänder oder digitale Speichermedien wie DVDs oder ähnliche verwendet werden. Auch das Ablegen einzelner Bilder im Speicher eines Computers gilt als Aufzeichnung im Sinne der Erfindung. Auch das Format ist nicht wesentlich. Es können unterschiedliche analoge oder digitale Filmformate, wie DV oder MPEG2 verwendet werden. Vorzugsweise werden bei der Verwendung von CCD-Kameras die beiden Bildinformationen auf ein digitales Videosystem, beispielsweise auf 2 Mini-DV-Rekorder, aufgezeichnet.
Bei einer bevorzugten Ausführungsform wird die Verbindung zwischen den Kameras 1, 2 und dem Videosystem erstmals auch über eine Funkstrecke realisiert. Dies ermöglicht die kabellose Übertragung der Videosignale zum Videosystem. Hierdurch wird vorteilhaft das ungehinderte Bewegen der Testperson als Fußgänger, als Radfahrer im freien Gelände oder auch auf bestimmten Arbeitseinsätzen, wie. z.B. auf Gerüsten oder Baustellen ermöglicht.
Wesentlich ist, daß die beiden Videosignale synchronisiert sind, d.h., daß für jedes Einzelbild des Augenvideos das entsprechende Einzelbild des Blickfeldvideos gefunden werden kann und umgekehrt. Die Synchronisierung kann mit einem periodischen Signalgenerator und Time-Code erfolgen. Vorzugsweise wird das Aufzeichnungsverfahren mit einem Tonpuls synchronisiert, der auf den jeweiligen Audiospuren mit aufgezeichnet wird. Dieses Verfahren ermöglicht es, gleichzeitig auch andere externen Geräte, wie z.B. UDS-Datenschreiber, GPS-Systeme o.ä. mit zu synchronisieren um auch weitere technische und medizinische Kenngrößen wie die aktuelle genaue geographische Position oder auch Herz- bzw. Pulsfrequenz, Hautwiderstand etc. der Testperson direkt mit dem Blickverhalten in Abstimmung bringen zu können. Die Synchronisierung ist für die spätere erfindungsgemäße Bearbeitung bzw. Auswertung der beiden Videosignale unabdinglich.

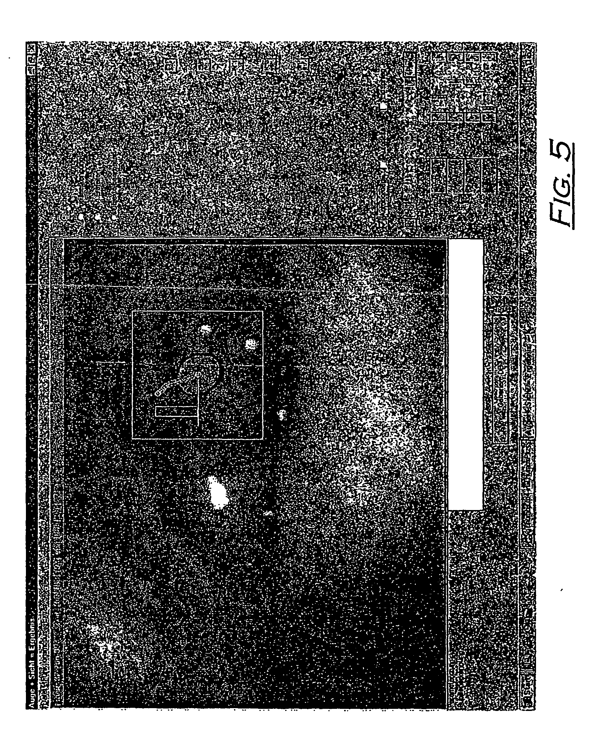

Bei dem erfindungsgemäßen Verfahren werden die genauen Koordinaten (xa,ya) des Pupillenmittelpunktes im Augenvideo durch ein Bilderkennungsprogramm ermittelt. Hierbei werden die Pupillenkoordinaten (xa,ya) für jedes Einzelbild des Augenvideos ermittelt. Die Pupillenkoordinaten (xa,ya) in einem Einzelbild des Augenvideos sind in Fig. 3 skizziert.
Die Ermittlung der Pupillenkoordinaten (xa,ya) erfolgt vorzugsweise automatisch mit einem Bilderkennungsprogramm. Hierfür werden für jedes Einzelbild des Augenvideos die Kontraste der Pupille zum Umfeld registriert und alle Punkte des Einzelbilds, die dunkler als ein eingestellter Dunkelheitsgrad sind, gesucht. Mit diesen Punkten wird eine Dunkelfläche voll erfaßt und abgegrenzt, und daraufhin automatisch der Schwerpunkt dieser Dunkelfläche ermittelt. Da die Dunkelfläche der Pupille der Testperson entspricht, stellt der Schwerpunkt der Dunkelfläche den Pupillenmittelpunkt dar.
Vorzugsweise bietet das Bilderkennungsprogramm Einstellungsvarianten für die entsprechenden Kontraste und den Dunkelheitsgrad, sodaß die höchstmögliche Genauigkeitsstufe für alle Einzelbilder erreicht werden kann. Für jedes Einzelbild kann somit für unterschiedliche Belichtungsverhältnisse der jeweils beste Kontrast in Form einer Grauwertschwelle sichergestellt werden, was insgesamt eine einwandfreie sichere Bestimmung der Pupillenkoordinaten (xa,ya) möglich macht.
Die Grauwertschwelle ist jener Wert, der z.B. bei digitalisierter Form zwischen 1 und 256 liegt, und den prozentuellen Anteil von Schwarz bzw. Weiß an einem Bildpunkt definiert. Der höchste erreichbare Wert entspricht einem vollen Schwarz, der niedrigste Wert dem Weiß. Da die Pupille bei der Aufzeichnung nie den vollen Schwarzwert erreicht, ist ein Wert zu definieren, der - zumindest für dieses Bild - dem real vorhandenen Pupillengrau entspricht. Der Schwellwert scheidet alle Bildpunkte aus, die heller als der definierte Grauwert sind, alle dunkleren Bereiche werden zur Schwerpunktsfindung herangezogen. Drei Parameter ermöglichen es, die Schwellendefinition zu optimieren. Da sich bei den Versuchen innerhalb einer Sequenz die Belichtungsverhältnisse oft sehr stark verändern, ist diese Schwellwertdefinition vorzugsweise auch für jedes Bild einzeln möglich. Alle Einstellungen können entsprechend den hohen Anforderungen für jedes Bild der Sequenz in einer Datei abgelegt werden. Durch das erfindungsgemäße Verfahren ist die besonders hohe Genauigkeit bei der Zuordnung der Pupillenkoordinaten (xa,ya) an das Blickfeld möglich. Die jeweilige Genauigkeitsstufe kann beispielsweise in Form einer Staffel visualisiert werden. In diesem Fall wird die Güte der ermittelten Fläche im Auswerteprogramm mittels der Staffel dargestellt, wobei es zunächst wichtig ist, daß ein positiver Wert erreicht wird. Negative Werte haben zur Folge, daß das jeweilige Bild ausgesondert wird. Je höher der positive Wert ausfällt, umso exakter können helle (Umfeld) von dunklen (Pupille) Bereichen unterschieden werden.
Zusätzlich kann zur genaueren Lokalisation des Pupillenmittelpunktes ein Infrarotfilter vor der Kamera vorgesehen sein. Durch diesen werden die Kontraste im Augenvideo verstärkt. Der IR Filter hat zwei wichtige Funktionen: Erstens erfolgt die Beleuchtung des Auges mit Infrarot Leuchtdioden (LED), die auch bei absoluter Dunkelheit gute Kontraste für die Augenkamera und für die weitere Bearbeitung gewährleisten. Der Filter hat die Aufgabe, das von den LED emittierte Licht auf den Kamerachip durchzulassen, alle anderen Spektralbereiche des Lichtes werden entsprechend der Filterdurchlaßkurve gedämpft. Zweitens sind die vom Sonnenlicht verursachten Reflexionen auf der Pupille, die sich massiv negativ auf die Schwerpunktsfindung auswirken, hauptsächlich im blauen Spektralbereich vorhanden. Auch hier hat der Filter die Aufgabe die Reflexionen auf der Pupille, die durch das Sonnenlicht verursacht werden, zu reduzieren.
Bei einer weiteren vorteilhaften Ausführung des erfindungsgemäßen Verfahrens erfolgt nach der automatischen Ermittlung der Pupillenkoordinaten (xa,ya) zusätzlich eine manuelle Kontrolle. Ein Bearbeiter kann bei einer fehlerhaften automatischen Erkennung (die beispielsweise bei plötzlichen Lichtreflexen auf der Augenoberfläche etc. auftreten) die Bildverarbeitungsparameter manuell ändern. Auch ist es möglich, die Pupillenkoordinaten (xa,ya) unmittelbar zu korrigieren. Eine Benutzeroberfläche für die erfindungsgemäße rechnerische Lokalisation des Pupillenmittelpunktes und die manuelle Kontrolle ist in Fig. 5 gezeigt.
Man erhält insgesamt für jedes Einzelbild des Augenvideos die Pupillenkoordinaten (xa,ya) beispielsweise in Form eines kartesischen Wertepaares. Natürlich können auch andere Koordinatensysteme, wie Polarkoordinaten etc. zum Einsatz kommen.

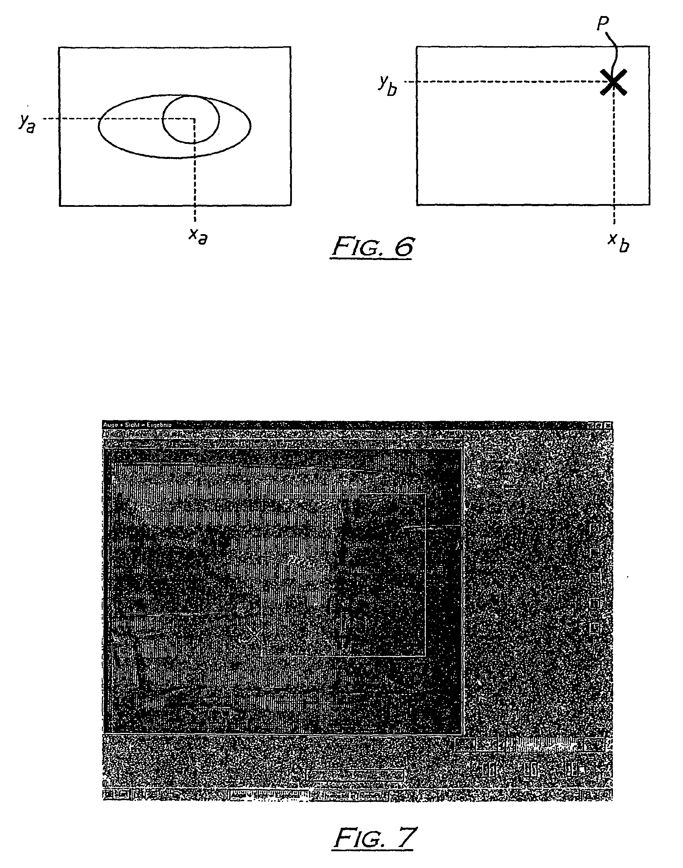

Da beide Kameras 1, 2 starr mit dem Kopf der Testperson verbunden sind, entspricht einer bestimmten Position der Pupille bzw. des Pupillenmittelpunktes im Augenvideo stets ein genau definierter Blickpunkt B im Blickfeldvideo. Aus dem Augenvideo und dem Blickfeldvideo kann somit berechnet werden, auf welchen Punkt die Testperson genau blickt.
Für die Zuordnung der Pupillenkoordinaten (xa,ya) zu den Koordinaten (xb,yb) des entsprechenden Blickpunktes B, d.h. des Punktes, den die Testperson fixiert, auf dem Blickfeldvideo muß zunächst die Korrelationsfunktion K zwischen diesen beiden Koordinatenpaaren (xa,ya) und (xb,yb) ermittelt werden. Die Korrelation zwischen Pupillenkoordinaten (xa,ya) und Blickpunkt B auf dem Blickfeldvideo erfolgt über eine Versuchsserie. Hierbei fixiert die Testperson der Reihe nach bestimmte vorgegebene Passpunkte P. Die Korrelationsfunktion K zwischen Pupillenkoordinaten (xa,ya) und den Koordinaten (xb,yb) im Blickfeldvideo wird anhand der hierbei gemessenen Daten erstellt.
Beim erfindungsgemäßen Verfahren wird die Korrelationsfunktion K zwischen Pupillenkoordinaten (xa,ya) auf dem Augenvideo und den Koordinaten (xb,yb) des entsprechenden Blickpunktes B auf dem Blickfeldvideo vorzugsweise automatisch ermittelt. Hierfür werden zunächst eine oder mehrere Musterblicksequenzen der Testperson auf einen oder mehrere bestimmte vorgegebene Passpunkte P aufgenommen. Unter Musterblicksequenz ist eine Blicksequenz zu verstehen, welche lediglich für die Kalibrierung aufgenommen wird, und bei der die Testperson auf vorgegebene Passpunkte P blickt.
Beispielsweise kann ein bestimmter Passpunkt P auf einer Wand markiert sein. Um einen bestmöglichen Kontrast zu erhalten, kann beispielsweise eine schwarze Markierung auf einer sonst weißen Oberfläche als Passpunkt P gewählt werden. Der Passpunkt P ist in der Regel ein Kreuz oder ein Leuchtpunkt oder ähnliches. Die Testperson wird angewiesen, diesen Passpunkt P zu fixieren, wobei das Blickfeld und das Auge der Testperson durch die beiden Kameras 1, 2 aufgenommen werden. Es können auf diese Weise mehrere Passpunkte P definiert werden. Auch ist es möglich, lediglich einen Passpunkt P zu markieren und die Testperson anzuweisen, bei Fixierung dieses Passpunktes P unterschiedliche Bewegungen mit dem Kopf auszuführen. Zwei Einzelbilder der auf diese Art erhaltenen Augen und Blickfeldvideos sind in Fig. 6 skizziert.
Da der Blickpunkt B auf dem so aufgenommenen Blickfeldvideo der Musterblicksequenz durch den bekannten Passpunkt P gegeben sind, kann in einem nächsten Schritt die Korrelationsfunktion K zwischen den Pupillenkoordinaten (xa,ya) auf dem Augenvideo und den Koordinaten (xb,yb) des entsprechenden Blickpunktes B auf dem Blickfeldvideo ermittelt werden.
Hierfür werden für jedes Einzelbild im Augenvideo die Pupillenkoordinaten (xa,ya) im Augenvideo gemäß dem oben beschriebenen Verfahren ermittelt.
Weiters werden die Koordinaten (xb,yb) des Passpunktes P im entsprechenden Einzelbild auf dem Blickfeldvideo ermittelt. Dies erfolgt bevorzugt mit einem Bilderkennungsverfahren, welches auf dem Blickfeldvideo die Koordinaten (xb,yb) des durch seinen Kontrast eindeutig erkennbaren Passpunktes P ermittelt.
Es ist aber auch möglich, die Koordinaten (xb,yb) des Passpunktes P im Blickfeldvideo jeweils für jedes Einzelbild händisch, beispielsweise durch Mausklickverfahren, zu bestimmen. Dies erlaubt eine Auswertung der Musterblicksequenz auch bei schwierigen Bedingungen im Gelände, bei denen eine automatische Bestimmung der Koordinaten (xb,yb) des Passpunktes P, beispielsweise wegen eines zu uneinheitlichen Hintergrundes, nicht möglich ist.
Somit können die Pupillenkoordinaten (xa,ya) im Einzelbild des Augenvideo den Koordinaten (xb,yb) des Passpunktes P im entsprechenden Einzelbild des Blickfeldvideos zugeordnet werden.
Die entsprechenden Koordinaten im Augen- und im Blickfeldvideo werden für jedes Einzelbild der Musterblicksequenz ermittelt und abgespeichert.

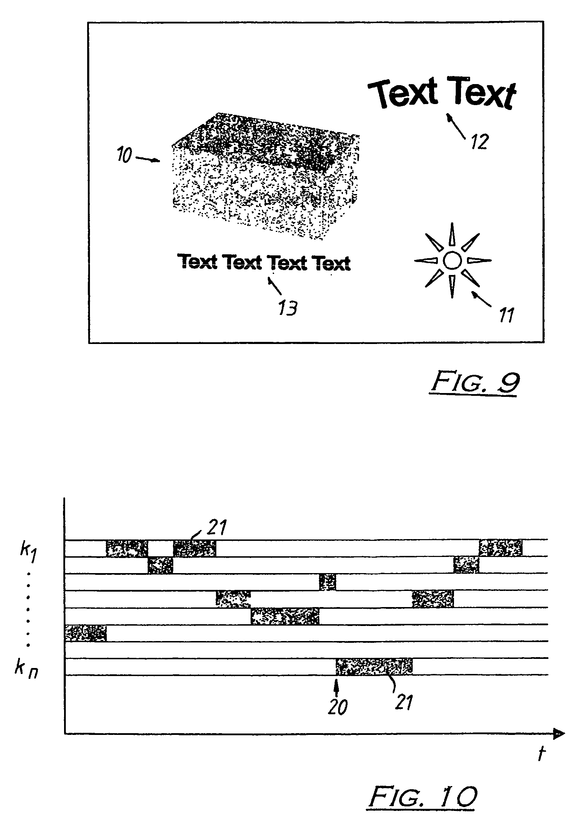

Aus allen so gewonnenen Datensätzen werden vorzugsweise durch quadratische Regression die Pupillenkoordinaten (xa,ya) auf dem Augenvideo und die Koordinaten (xb,yb) des entsprechenden Blickpunktes B auf dem Blickfeldvideo korreliert. Natürlich sind auch andere Verfahren wie lineare Regression oder stochastische Modelle für die Korrelation möglich. Eine Benutzeroberfläche für die erfindungsgemäße Ermittlung der Korrelationsfunktion K ist in Fig. 7 dargestellt.
Man erhält eine Korrelationsfunktion K: (xa,ya) -> (xb,yb), welche einem bestimmten Satz von Pupillenkoordinaten (xa,ya) auf dem Augenvideo eindeutig die entsprechenden Koordinaten (xb,yb) des Blickpunktes B im Blickfeldvideo zuordnet.
Für eine größtmögliche Genauigkeit der Korrelationsfunktion K sollten zumindest 25 unterschiedliche Positionen des Passpunktes P verwendeten werden. Ab etwa 100 unterschiedlichen Passpunktpositionen nimmt die erzielte Genauigkeit kaum mehr zu, sodaß eine weitere Erhöhung der Zahl der Passpunktpositionen nicht mehr sinnvoll ist. Vorzugsweise werden somit zwischen 25 und 100 Passpunktpositionen verwendet.
Mit der so ermittelten Korrelationsfunktion K können alle weiteren Videosequenzen der gleichen Versuchsserie, d.h. bei denen es keine Änderungen bezüglich der Kamerapositionen auf dem Kopf der Testperson gibt, errechnet werden.
Durch die numerische Korrelation der beiden Koordinatenpaare können nichtlineare Zusammenhänge erfaßt werden. Dies bietet einen bedeutenden Vorteil gegenüber bekannten Systemen, bei denen die beiden Videosignale einfach übereinandergelegt werden. Das Ergebnis bei diesen bekannten Verfahren ist ein Ergebnisvideo, welches das Blickfeld der Testperson zeigt. Über das Blickfeld ist die Pupille der Testperson überlagert, so daß ersichtlich ist, welcher Punkt von der Testperson fixiert wird. Diese bekannten Verfahren sind allerdings sehr ungenau. Geometrische Verschiebungen zwischen der Position der Pupille im Augenvideo und dem Blickpunkt müssen videotechnisch kompensiert werden. Beim erfindungsgemäßen Verfahren müssen derlei geometrische Faktoren nicht berücksichtigt werden. Die korrekte Zuordnung der Pupillenkoordinaten (xa,ya) zum Blickpunkt B erfolgt über die Korrelationsfunktion K.

Nach der Kalibrierung des Blickerfassungssystems können nun einzelne Blicksequenzen erfaßt und analysiert werden. Nach erfolgter Ermittlung der Korrelationsfunktion K werden hierbei für jedes Einzelbild aus den Pupillenkoordinaten (xa,ya) auf dem Augenvideo die Koordinaten (xb,yb) des entsprechenden Blickpunktes B auf dem Blickfeldvideo extrapoliert.
Die Zusammenführung des Augenvideos und des Blickfeldvideos in einem Ergebnisvideo erfolgt softwaretechnisch so, daß die errechneten Blickpunkte B als Mittelpunkte der Blickzuwendungen auf dem Blickfeldvideo positioniert werden. Durch die erfindungsgemäße rechnerische Ermittlung der Koordinaten (xb,yb) der Blickpunkte B ist die besonders genaue Darstellung des Mittelpunktes der Blickzuwendung möglich.

Der Blickpunkt B kann auf dem Ergebnisvideo genau eingezeichnet werden. Vorzugsweise wird der Blickpunkt B auf dem Ergebnisvideo durch eine visuell gut sichtbare Markierung, beispielsweise durch ein Kreuz, angedeutet. Fig. 8 zeigt ein Einzelbild eines so erzeugten Ergebnisvideos.
In einem nächsten Schritt kann das Ergebnisvideo in einer eigenen Datei abgespeichert werden, wobei übliche Videokomprimierungsverfahren zur Anwendung kommen können.

Das erfindungsgemäße Verfahren kann vollständig automatisiert durchgeführt werden. In diesem Fall ist eine händische Kontrolle der Pupillenkoordinaten (xa,ya) nicht vorgesehen. Diese werden unmittelbar über das Bilderkennungsprogramm aus den Einzelbildern des Augenvideos ermittelt. Desweiteren werden die Koordinaten (xb,yb) der Passpunkte P während des Kalibrierungsschrittes automatisch ermittelt. Somit ist das weiter oben beschriebenes Abspeichern des gesamten Augenvideos nicht mehr unbedingt notwendig.
Bei einer bevorzugten Ausführungsform der Erfindung bietet die Verarbeitungssoftware die weitere vorteilhafte Möglichkeit, das Blickfeld in unterschiedliche Einheiten, sogenannten Kategorien ki, aufzugliedern. Die jeweiligen Bildinhalte können zu frei wählbaren und definierbaren Kategorien ki zugeordnet werden.
Diese Einteilung in Kategorien ki ist nicht nur für das erfindungsgemäße, sondern auch für andere Verfahren zur Erfassung, Auswertung und Analyse von Blicksequenzen vorteilhaft, bei denen das Blickfeld der Testperson durch eine erste Kamera 1 erfaßt wird und für jedes Einzelbild des Blickfeldvideos die Koordinaten (xb,yb) des entsprechenden Blickpunktes B auf dem Blickfeldvideo ermittelt werden.
Die Kategorien ki k1..kn können statische oder bewegliche Elemente des Blickfeldes sein. Beispielsweise kann bei einem Flugsimulator eine Kategorie ki durch ein fremdes Flugzeug dargestellt sein, eine weitere durch die Höhenanzeige etc. Bei Werbeplakaten kann eine Kategorie ki durch ein bestimmtes Logo, eine andere durch einen bestimmten Text gegeben sein usw. Beispielsweise enthält das in Fig. 9 skizzierte Bild bzw. Schild oder Werbeplakat eine bildliche Darstellung 10, ein Logo 11 einen Titel 12 und einen erläuternden Werbetext 13. Bei der Analyse der Blicksequenzen auf dieses Standbild kann jedem dieser Elemente eine eigene Kategorie ki zugeordnet werden. In Fig. 11 ist der bildlichen Darstellung 10 die Kategorie k1, dem Logo 11 die Kategorie k2, dem Titel 12 die Kategorie k3 und dem erläuternden Werbetext 13 die Kategorie k4 zugeordnet.
Die Zuordnung der einzelnen Blickpunkte zu den einzelnen Kategorien ki kann automatisch erfolgen. Wesentlich ist, daß mehrere Kategorien ki definiert werden und der Blickpunkt B für jedes Einzelbild einer Kategorie ki zugeordnet wird, indem für jedes Einzelbild überprüft wird, im Bereich welcher Kategorie ki der Blickpunkt B liegt. Liegt der Blickpunkt B in keiner der definierten Kategorien ki, kann er einer zusätzlichen Nullkategorie zugeordnet werden.

Beispielsweise können im Fall eines statischen Bildes einzelne Kategorien ki anhand der Häufungen der Blickpunkte definiert werden. Somit erfolgt die Einteilung in Kategorien ki voll automatisch. Bei beweglichen Bildern, wie im Fall des genannten Flugsimulators, können die beweglichen Objekte durch spezielle Bilderkennungssoftware erkannt und lokalisiert werden. Auch ist eine Kommunikation zwischen dem Flugsimulator und dem Blickerkennungssystem möglich, bei dem die Koordinaten (xb,yb) der einzelnen Flugzeuge, bzw. Kategorien ki unmittelbar eingelesen werden können.
Andererseits ist auch die händische Zuordnung der Blickpunkte zu den einzelnen Kategorien ki für jedes Einzelbild möglich.

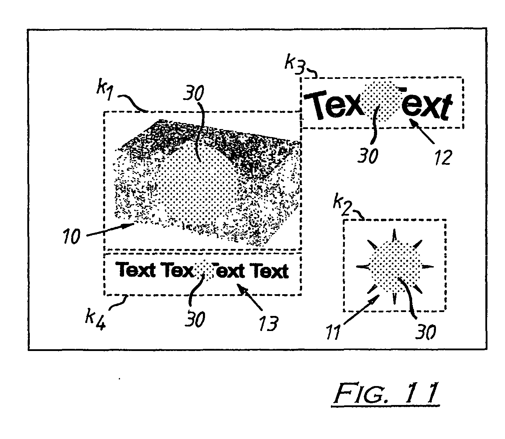

Das erfindungsgemäße Verfahren erlaubt durch das Vorliegen der Daten in digitalisierter Form eine weitere Aufbereitung der Daten, insbesondere der Zuordnung der einzelnen Blickpunkte B zu den unterschiedlichen Kategorien ki, die die Analyse derselben stark vereinfacht.
Bei einer vorteilhaften Ausführung des Verfahrens kann vorgesehen sein, daß zur komprimierten Darstellung der zeitlichen Abfolge der Zugehörigkeit des Blickpunktes B zu einer bestimmten Kategorie ki einer Blicksequenz ein Diagramm erstellt wird, welches zwei Achsen aufweist, wobei eine Achse einer Zeitachse entspricht und die andere Achse den einzelnen Kategorien ki entspricht. Hierfür wird für jedes Einzelbild ermittelt, in welcher Kategorie ki sich der Blickpunkt B befindet und in dem Diagramm an der dem Zeitpunkt des Einzelbildes entsprechenden Höhe der Zeitachse und der der Kategorie ki entsprechenden Höhe der anderen Achse eine visuelle Marke 20, beispielsweise ein Strich, eingetragen.
Das auf diese Weise erzeugte sogenannte lineare Blickband, welches in Fig. 10 dargestellt ist, ist eine Darstellungsform, bei der auf einer Achse die Zeit, in Sekunden oder in Einzelbildern, dargestellt wird und auf der anderen Achse die frei zu wählenden Kategorien ki aufgetragen werden. Durch die Eintragung einzelner visueller Marken 20 bzw. einzelner Striche für jedes Einzelbild entsprechend der Zuordnung des Blickpunktes B zu einer der aufgetragenen Kategorien ki ergibt sich eine Balkendarstellung, bei der jeder Balken 21 den Beginn und das Ende einer Blickzuwendung zu einer bestimmten Kategorie ki angibt. Der zeitliche Ablauf des Festhaltens des Blickes der Testperson an bestimmten Kategorien ki wird somit als Diagramm über der Zeit klar ersichtlich. Selbstverständlich können auch andere visuelle Marken 20 wie Punkte, Kreuze etc. verwendet werden.
Natürlich ist es auch möglich, in dem linearen Blickband Zusatzinformationen, die über Videoauswertungen hinausgehen, einzutragen. Dazu zählen Informationen, die den Handlungsablauf beschreiben, wie z.B. Veränderungen im Straßenraum, aber auch Zusatzinformationen aus anderen Datenquellen wie beispielsweise UDS-Datenschreiber, (Geschwindigkeit, Richtung), oder auch in weiterer Folge humanphysiologische Daten des Pulsschlages oder Lidschlages, der Körpertemperatur usw.
Im linearen Blickband können, entsprechend den jeweiligen Zeitabschnitten, die Kategorien ki durch Balken 21, die in verschiedenen Farben die Kategorien ki symbolisieren, zentral dargestellt werden. Es können innerhalb dieser einzelnen Kategorien ki Zusammenfassungen getroffen werden, die dann farblich inhaltlich unterschieden werden, wie z.B. örtliche Unterschiede oder inhaltliche Unterschiede wie Links/Rechts oder Vorne/ Hinten. Das lineare Blickband ist grundsätzlich nicht in seiner Länge oder in seinen Kategorien ki begrenzt. Um eine anschauliche Darstellungsform zu ermöglichen, ist es jedoch sinnvoll, Ausschnitte inhaltlich zusammenzufassen.

Bei einer weiteren vorteilhaften Darstellungsform der Vorgänge bei einer Blicksequenz auf ein Standbild, d.h. auf ein nicht bewegtes Bild, wie z.B. eine Werbetafel, können die Verweildauern des Blickpunktes B auf unterschiedlichen Kategorien ki grafisch dargestellt werden. Hierfür wird für jede Kategorie ki ermittelt, bei welchen Einzelbildern der Blickpunkt B im Bereich dieser Kategorie ki liegt. Die Zeiten, für die der Blickpunkt B im Bereich dieser Kategorie ki liegt, werden addiert, wobei die so ermittelte Summe der Verweildauer des Blickpunktes B auf dieser Kategorie ki entspricht. Aus den unterschiedlichen Verweildauern wird anschließend eine Grafik bzw. ein Bild erstellt, welches im wesentlichen dem Standbild entspricht, wobei aber jede Verweildauer auf den einzelnen Kategorien ki jeweils durch ein grafisches Element 30 dargestellt ist, welches im Bereich der Kategorie ki plaziert ist und zumindest einen Parameter - Durchmesser, Farbe o.ä. - aufweist, der der Verweildauer proportional ist. Beispielsweise können unterschiedliche Verweildauern durch grafische Elemente 30 unterschiedlicher Größe (beispielsweise ist der Umfang des grafischen Elements 30 umso größer, je länger die Verweildauer ist) oder unterschiedlicher Farbe dargestellt werden. Fig. 11 zeigt ein aus dem Werbeplakat von Fig. 9 gewonnenes Diagramm, in welchem die Verweildauern auf den einzelnen Kategorien ki durch grafische Elemente 30 angezeigt sind, welche als Punktwolken unterschiedlichen Durchmessers ausgeführt sind. Weiters sind in Fig. 11 die Abgrenzungen der vier definierten Kategorien k1 bis k4 durch gestrichelte Linien angedeutet.
Dieses erfindungsgemäße Diagramm zur komprimierten Darstellung der Verweildauer des Blickpunktes B auf einer bestimmten Kategorie ki einer Blicksequenz auf ein Standbild zeichnet sich somit dadurch aus, daß im Bereich jeder Kategorie ki ein grafisches Element angeordnet ist, welches der Verweildauer proportional ist. Die Häufigkeit des Festhaltens des Blickpunktes B auf einer bestimmten Kategorie ki wird in dem erfindungsgemäßen Diagramm durch ein der Verweildauer proportionales grafisches Elemente 30, etwa einen unterschiedlich großen Punkt, angezeigt.
Durch dieses Diagramm können die Häufigkeiten und die Zeitbedarfsanalyse besonders deutlich visualisiert werden. Das erfindungsgemäße Diagramm ermöglicht eine qualitative hochkarätige und anschauliche Analyse der Blickzuwendungshäufigkeiten.

Bei einer weiteren Darstellungsform, der sogenannten Blickfeldskizze, werden die Blickabfolgen in der Reihenfolge, wie sie im Ergebnisvideo in Erscheinung treten, aufgetragen. Dabei können perspektivische Darstellungen oder entzerrte Darstellungen der von der Testperson betrachteten Szene zur Anwendung gelangen, wie z.B. Aufriss- oder Grundrissskizzen. Bei Aufrissskizzen können in einem Screenshot in parallel erstellten Fotos, aber auch in Skizzen, die Blickabfolgen grafisch erstellt werden. Für Grundrissskizzen können Lagepläne, stilisierte Pläne, aber auch Übersichtsdarstellungen für konkrete Inhalte herangezogen werden. In der Blickfeldskizze wird jedem Blickpunkt, bzw. jeder Fixation ein Pfeil zugeordnet, unabhängig von der Fixationslänge. Die Fixationslängen bzw. thematischen Zusammenhänge können farblich, aber auch durch Zusatzinformationen näher beschrieben werden. Als Zusatzinformationen sind Numerierungen bzw. Erklärungstexte möglich. Ergänzend können auch zeitliche Zusammenhänge und unterschiedliche Farbskalierungen dargestellt werden, d.h. es können Zeiten gebündelt und in einer konkreten Farbe mit entsprechender Legende ausgewiesen werden.

Das erfindungsgemäße Verfahren eignet sich besonders für die Analyse des Blickverhaltens verschiedener Testpersonen in einer selben, vorgegebenen Untersuchungssituation. Hierfür wird die Untersuchungssituation aufgezeichnet und anschließend unterschiedlichen Testpersonen vorgespielt, wobei deren Blickverhalten analysiert wird. Dies ist beispielsweise für die Untersuchung einer bestimmten gefährlichen Stelle im Straßenverkehr vorteilhaft. Für die selbe Verkehrssituation kann das Blickverhalten unterschiedlicher Testpersonen, beispielsweise altersabhängig oder abhängig davon, ob die Testperson unter dem Einfluß bestimmter Medikamente, Drogen oder Alkohol steht, untersucht werden. Es können aber auch andere Untersuchungssituationen wie Arbeitseinsätze auf Baustellen oder in Kraftwerksleitstellen oder -sicherungen oder allgemeine Situationen in Mensch-Maschine Systemen aufgezeichnet werden, wobei später das Blickverhalten unterschiedlicher Testpersonen in diesen Untersuchungssituationen analysiert werden kann. Die Aufzeichnung der Untersuchungssituation erfolgt beispielsweise mit einer einfachen Videokamera, wobei diese Aufzeichnung den Testpersonen z.B. über einen Videoprojektor vorgespielt werden kann.

## Patentansprüche

1. Verfahren zur Erfassung, Auswertung und Analyse von Blicksequenzen einer Testperson mittels eines Blickerfassungssystems,
- wobei das Blickfeld der Testperson durch eine nach vorne gerichtete und starr mit dem Kopf der Testperson verbundene erste Kamera (1) erfaßt und in einem Blickfeldvideo aufgezeichnet wird,
- die Bewegung der Pupille der Testperson durch eine zweite Kamera (2), die ebenfalls starr mit dem Kopf der Testperson verbunden ist, erfaßt und in einem Augenvideo aufgezeichnet wird
- und das Augenvideo und das Blickfeldvideo auf ein Videosystem aufgezeichnet und zeitlich synchronisiert werden,
- wobei für jedes Einzelbild des Augenvideos die Pupillenkoordinaten (xa,ya) ermittelt werden,
- die Korrelationsfunktion (K) zwischen Pupillenkoordinaten (xa,ya) auf dem Augenvideo und den Koordinaten (xb,yb) des entsprechenden Blickpunktes (B), d.h. des Punktes, den die Testperson fixiert, auf dem Blickfeldvideo ermittelt wird
- und nach erfolgter Ermittlung der Korrelationsfunktion (K) für jedes Einzelbild aus den Pupillenkoordinaten (xa,ya) auf dem Augenvideo die Koordinaten (xb,yb) des entsprechenden Blickpunktes (B) auf dem Blickfeldvideo extrapoliert werden,
**dadurch gekennzeichnet, daß**
zur Ermittlung der Pupillenkoordinaten (xa,ya) für jedes Einzelbild des Augenvideos mit einem Bilderkennungsprogramm automatisch
- die Kontraste der Pupille zum Umfeld registriert werden,
- alle Punkte des Einzelbilds, die dunkler als ein eingestellter Dunkelheitsgrad sind, gesucht werden,
- mit diesen Punkten eine der Pupille entsprechende Dunkelfläche voll erfaßt und abgegrenzt wird, und
- der dem Pupillenmittelpunkt mit den Pupillenkoordinaten (xa,ya) entsprechende Schwerpunkt der Dunkelfläche ermittelt wird.

2. Verfahren nach Anspruch 1, **dadurch gekennzeichnet, daß** nach der automatischen Ermittlung der Pupillenkoordinaten (xa,ya) eine händische Korrektur der Pupillenkoordinaten (xa,ya) erfolgt.

3. Verfahren nach Anspruch 1 oder 2, **dadurch gekennzeichnet, dass** zur Ermittlung der Korrelationsfunktion (K)
- zunächst eine oder mehrere Musterblicksequenzen der Testperson auf einen oder mehrere bestimmte vorgegebene Passpunkte (P) aufgenommen werden
- und die Zuordnung der Pupillenkoordinaten (xa,ya) auf dem Augenvideo zu den Koordinaten (xb,yb) des Passpunktes (P) auf dem Blickfeldvideo ermittelt wird,
- indem für jedes Einzelbild im Augenvideo die Pupillenkoordinaten (xa,ya) im Augenvideo ermittelt werden,
- die Koordinaten (xb,yb) des Passpunktes (P) im entsprechenden Einzelbild auf dem Blickfeldvideo ermittelt werden,
- die Pupillenkoordinaten (xa,ya) im Einzelbild des Augenvideo den Koordinaten (xb,yb) des Passpunktes (P) im entsprechenden Einzelbild des Blickfeldvideo zugeordnet werden,
- dieser Datensatz abgespeichert wird,
- und aus allen Datensätzen, vorzugsweise durch quadratische Regression, die Pupillenkoordinaten (xa,ya) auf dem Augenvideo und die Koordinaten (xb,yb) des entsprechenden Blickpunktes (B) auf dem Blickfeldvideo korreliert werden.

4. Verfahren nach Anspruch 3, **dadurch gekennzeichnet, daß** die Koordinaten (xb,yb) des Passpunktes (P) auf jedem Einzelbild des Blickfeldvideos automatisch durch ein Bilderkennungsverfahren ermittelt werden.

5. Verfahren nach Anspruch 3, **dadurch gekennzeichnet, daß** die Koordinaten (xb,yb) des Passpunktes (P) auf jedem Einzelbild des Blickfeldvideos händisch mittels Mausklickverfahren ermittelt werden.

6. Verfahren nach einem der Ansprüche 3 bis 5, **dadurch gekennzeichnet, daß** die Musterblicksequenzen durch Drehen des Kopfes der Testperson bei Fixierung eines einzelnen Passpunktes (P) erhalten werden.

7. Verfahren nach einem der Ansprüche 3 bis 6, **dadurch gekennzeichnet, daß** zwischen 25 und 100 Positionen des Passpunktes (P) bzw. der Passpunkte (P) verwendet werden.

8. Verfahren nach einem der vorangegangenen Ansprüche, **dadurch gekenntzeichnet**, **daß** aus dem Blickfeldvideo und den ermittelten Koordinaten (xb,yb) des Blickpunktes (B) auf dem Blickfeldvideo ein Ergebnisvideo erzeugt wird, auf dem der Blickpunkt (B) durch eine visuell gut sichtbare Markierung, insbesondere durch ein Kreuz, angezeigt wird.

9. Verfahren nach einem der Ansprüche 1 bis 8, **dadurch gekennzeichnet, daß** mehrere Kategorien (ki) definiert werden und der Blickpunkt (B) für jedes Einzelbild einer Kategorie (ki) zugeordnet wird, indem für jedes Einzelbild überprüft wird, im Bereich welcher Kategorie (ki) der Blickpunkt (B) liegt.

10. Verfahren nach Anspruch 9, **dadurch gekennzeichnet, daß** zur komprimierten Darstellung der zeitlichen Abfolge der Zugehörigkeit des Blickpunktes (B) zu bestimmten Kategorien (ki) einer Blicksequenz ein Diagramm erstellt wird, welches zwei Achsen aufweist, wobei eine Achse einer Zeitachse entspricht und die andere Achse den einzelnen Kategorien (ki) entspricht, wobei für jedes Einzelbild ermittelt wird, in welcher Kategorie (ki) sich der Blickpunkt (B) befindet und in dem Diagramm an der dem Zeitpunkt des Einzelbildes entsprechenden Höhe der Zeitachse und der der Kategorie (ki) entsprechenden Höhe der anderen Achse eine visuelle Marke (20) eingetragen wird.

11. Verfahren nach Anspruch 9 oder 10, **dadurch gekennzeichnet, daß** zur komprimierten Darstellung der Verweildauer des Blickpunktes (B) auf bestimmten Kategorien (ki) einer Blicksequenz auf ein Standbild
- für jede Kategorie (ki) ermittelt wird, bei welchen Einzelbildern der Blickpunkt (B) in einer Kategorie (ki) liegt,
- die Verweildauer des Blickpunktes (B) auf dieser Kategorie (ki) durch Addition der Dauer dieser Einzelbilder ermittelt wird, und
- ein Bild erstellt wird, welches dem Standbild entspricht, in dem im Bereich jeder Kategorie (ki) ein grafisches Element (30) angeordnet ist, welches zumindest einen Parameter aufweist, der der Verweildauer proportional ist.

12. Verfahren nach Anspruch 9, 10 oder 11, **dadurch gekennzeichnet, daß** zur komprimierten Darstellung der Reihenfolge der Blickabfolgen eine perspektivische oder entzerrten Darstellungen der von der Testperson betrachteten Szene erstellt wird und in dieser jedem Blickpunkt (B) ein Pfeil zugeordnet wird.

13. Verfahren nach einem der Ansprüche 1 bis 12, **dadurch gekennzeichnet, daß** die Untersuchungssituation aufgezeichnet wird, und diese Aufzeichnung jeder Testperson vorgespielt wird, wobei deren Blicksequenzen mittels eines Blickerfassungssystems erfaßt und ausgewertet werden,
- wobei das Blickfeld der Testperson durch eine nach vorne gerichtete und starr mit dem Kopf der Testperson verbundene erste Kamera (1) erfaßt und in einem Blickfeldvideo aufgezeichnet wird,
- für jedes Einzelbild des Blickfeldvideos die Koordinaten (xb,yb) des entsprechenden Blickpunktes (B), d.h. des Punktes, den die Testperson fixiert, auf dem Blickfeldvideo ermittelt werden.

## Claims

1. Method for detecting, evaluating and analysing sequences of vision of a test person by means of a vision detection system,
- wherein the field of vision of the test person is detected by a first camera (1) directed to the front and rigidly connected to the head of the test person and is recorded in a field of vision video,
- the movement of the pupil of the test person is detected by a second camera (2) which is also rigidly connected to the head of the test person, and is recorded in an eye video.
- and the eye video and the field of vision video are recorded on a video system and are synchronised with respect to time,
- wherein the pupil coordinates (xa, ya) are determined for each individual image of the eye video,
- the correlation function (K) between pupil coordinates (xa, ya) on the eye video and the coordinates (xb, yb) of the corresponding point of vision (B), i.e. the point, which the test person fixes, on the field of vision video is determined,
- and once the correlation function (K) has been determined for each individual image, the coordinates (xb, yb) of the corresponding point of vision (B) on the field of vision video are extrapolated from the pupil coordinates (xa, ya) on the eye video,
**characterised in that** to determine the pupil coordinates (xa, ya) for each individual image of the eye video with an image recognition programme, automatically,
- the contrasts of the pupil to the surroundings are registered,
- all the points of the individual image, which are darker than an adjusted degree of darkness, are looked for,
- a dark area corresponding to the pupil is fully detected with these points and is delimited, and
- the focal point of the dark area corresponding to the centre point of the pupil with the pupil coordinates (xa, ya) is determined.

2. Method according to claim 1, **characterised in that**, after the automatic determination of the pupil coordinates (xa, ya), the pupil coordinates (xa, ya) are corrected manually.

3. Method according to claim 1 or 2, **characterised in that** to determine the correlation function (K)
- one or more sample sequences of vision of the test person are firstly recorded on one or more specific input control points (P),
- and the allocation of the pupil coordinates (xa, ya) on the eye video to the coordinates (xb, yb) of the control point (P) on the field of vision video is determined,
- **in that** the pupil coordinates (xa, ya) in the eye video are determined for each individual image in the eye video,
- the coordinates (xb, yb) of the control point (P) in the corresponding individual image on the field of vision video are determined,
- the pupil coordinates (xa, ya) in the individual image of the eye video are allocated to the coordinates (xb, yb) of the control point (P) in the corresponding individual image of the field of vision video,
- this data record is stored.
- and the pupil coordinates (xa, ya) on the eye video and the coordinates (xb, yb) of the corresponding point of vision (B) on the field of vision video are correlated from all the data records, preferably by quadratic regression.

4. Method according to claim 3, **characterised in that** the coordinates (xb, yb) of the control point (P) on each individual image of the field of vision video are determined automatically by an image recognition method.

5. Method according to claim 3, **characterised in that** the coordinates (xb, yb) of the control point (P) on each individual image of the field of vision video are determined manually by a mouse clicking method.

6. Method according to any one of claims 3 to 5, **characterised in that** the sample sequences of vision are obtained by turning the head of the test person when fixing an individual control point (P).

7. Method according to any one of claims 3 to 6, **characterised in that** between 25 and 100 positions of the control point (P) or control points (P) are used.

8. Method according to any one of the preceding claims, **characterised in that**, from the field of vision video and the determined coordinates (xb, yb) of the point of vision (B) on the field of vision video, a result video is produced, on which the point of vision (B) is displayed by a visually very conspicuous marking, in particular by a cross.

9. Method according to any one of claims 1 to 8, **characterised in that** a plurality of categories (ki) are defined and the point of vision (B) for each individual image is allocated to a category (ki), **in that**, for each individual image, a check is made as to in the region of which category (ki) the point of vision (B) is located.

10. Method according to claim 9, **characterised in that** for compressed representation of the time sequence of the association of the point of vision (B) with certain categories (ki), of a vision sequence, a diagram is set up, which has two axes, one axis corresponding to a time axis and the other axis to the individual categories (ki), wherein it is determined for each individual image in which category (ki) the point of vision (B) is located and a visual mark (20) is entered in the diagram at the level of the time axis corresponding to the instant of the individual image and at the level of the other axis corresponding to the category (ki).

11. Method according to claim 9 or 10, **characterised in that** for the compressed representation of the residence duration of the point of vision (B) at certain categories (ki) of a vision sequence, on a fixed image,
- for each category (ki) it is determined at which individual images the point of vision (B) is located in a category (ki),
- the residence duration of the point of vision (B) at this category (ki) is determined by addition of the duration of these individual images, and
- an image is set up, which corresponds to the fixed image, in which, in the region of each category (ki) a graphic element (30) is arranged, which has at least one parameter, which is proportional to the residence duration.

12. Method according to claim 9, 10 or 11, **characterised in that** for the compressed representation of the order of the vision sequences, a perspective or corrected representation of the scene observed by the test person is set up and an arrow is allocated therein to each point of vision (B).

13. Method according to any one of claims 1 to 12, **characterised in that** the investigation situation is recorded and this recording is played to each test person, their vision sequences being detected and evaluated by means of a vision detection system,
- wherein the field of vision of the test person is detected by a first camera (1) directed to the front and rigidly connected to the head of the test person and recorded in a field of vision video,
- for each individual image of the field of vision video, the coordinates (xb, yb) of the corresponding point of vision (B), i.e. the point, which the test person fixes, on the field of vision video are determined.

## Revendications

1. Méthode permettant d'obtenir, d'interpréter et d'analyser des séquences de vision d'une personne testée au moyen d'un système de détection du regard,
- dans lequel le champ de vision de la personne testée est capté par une première caméra (1) dirigée vers l'avant et fixée de façon rigide à la tête de la personne testée et enregistré dans une vidéo du champ de vision,
- le mouvement de la pupille der la personne testée est capté par une deuxième caméra (2), également fixée de façon rigide à la tête de la personne testée et enregistrée dans une vidéo des yeux,
- et la vidéo des yeux et la vidéo du champ de vision sont enregistrées dans un système vidéo et synchronisées dans le temps,
- les coordonnées des pupilles (xa,ya) étant déterminées pour chaque image de la vidéo des yeux,
- la fonction de corrélation (K) entre les coordonnées des pupilles (xa,ya) dans la vidéo des yeux et les coordonnées (xb,yb) du point regardé (B) correspondant, c'est-à-dire du point que la personne testée fixe, est déterminée dans la vidéo du champ de vision
- et après la détermination de la fonction de corrélation (K) pour chaque image, les coordonnées des pupilles (xa,ya) dans la vidéo des yeux servent à extrapoler les coordonnées (xb,yb) du point regardé (B) correspondant dans la vidéo du champ de vision,
**caractérisée en ce que** pour déterminer les coordonnées des pupilles (xa,ya) pour chaque image de la vidéo des yeux, un programme de reconnaissance du regard effectue automatiquement les opérations suivantes :
- enregistrer les contrastes de la pupille par rapport à l'environnement,
- rechercher tous les points de l'image plus sombres d'un niveau de teinte sombre donné,
- détecter à l'aide de ces points l'ensemble d'une surface sombre correspondant à la pupille et délimiter celle-ci, et
- déterminer le barycentre de la surface sombre correspondant au centre de la pupille à l'aide des coordonnées des pupilles (xa,ya).

2. Méthode selon la revendication 1, **caractérisée en ce qu'**après la détermination automatique des coordonnées des pupilles (xa,ya), les coordonnées des pupilles (xa,ya) font l'objet d'une correction manuelle.

3. Méthode selon la revendication 1 ou 2, **caractérisée en ce que** pour déterminer la fonction de corrélation (K) :
- une ou plusieurs séquences types de vision de la personne testée sont enregistrées sur un ou plusieurs points de passage (P) prédéterminés,
- et l'association des coordonnées des pupilles (xa,ya) dans la vidéo des yeux aux coordonnées (xb,yb) du point de passage (P) sur la vidéo du champ de vision est déterminée,
- en déterminant pour chaque image dans la vidéo des yeux les coordonnées des pupilles (xa,ya) dans la vidéo des yeux,
- en déterminant les coordonnées (xb,yb) du point de passage (P) dans l'image correspondante dans la vidéo du champ de vision,
- en associant les coordonnées des pupilles (xa,ya) dans l'image de la vidéo des yeux aux coordonnées (xb,yb) du point de passage (P) dans l'image correspondante de la vidéo du champ de vision,
- en enregistrant cet ensemble de données,
- et à partir de ces ensembles de données, les coordonnées des pupilles (xa,ya) dans la vidéo des yeux et les coordonnées (xb,yb) du point regardé (B) correspondant dans la vidéo du champ de vision sont corrélées, de préférence par régression quadratique.

4. Méthode selon la revendication 3, **caractérisée en ce que** les coordonnées (xb,yb) du point de passage (P) dans chaque image de la vidéo du champ de vision sont automatiquement déterminées par un Méthode de reconnaissance d'images.

5. Méthode selon la revendication 3, **caractérisée en ce que** les coordonnées (xb,yb) du point de passage (P) dans chaque image de la vidéo du champ de vision sont déterminées manuellement à l'aide de clics d'une souris.

6. Méthode selon l'une des revendications 3 à 5, **caractérisée en ce que** les séquences types de vision sont obtenues en faisant tourner la tête de la personne testée tout en lui faisant fixer un seul point de passage (P).

7. Méthode selon l'une des revendications 3 à 6, **caractérisée en ce que** 25 à 100 positions du point de passage (P) ou des points de passage (P) sont utilisées.

8. Méthode selon l'une des revendications précédentes, **caractérisée en ce qu'**à partir de la vidéo du champ de vision et des coordonnées déterminées (xb,yb) du point regardé (B) dans la vidéo du champ de vision, on produit une vidéo de résultat dans laquelle le point regardé (B) est indiqué par un marquage bien visible, en particulier par une croix.

9. Méthode selon l'une des revendications 1 à 8, **caractérisée en ce que** plusieurs catégories (ki) sont définies et le point regardé (B) est associé pour chaque image à une catégorie (ki) en vérifiant pour chaque image dans quelle catégorie (ki) se trouve le point regardé (B).

10. Méthode selon la revendication 9, **caractérisée en ce qu'**en vue d'une représentation compressée de la séquence dans le temps de l'affectation du point regardé (B) à certaines catégories (ki) d'une séquence de vision, un diagramme à deux axes est créé dont un axe correspond à un axe de temps et l'autre axe aux différentes catégories (ki), et on détermine pour chaque image dans quelle catégorie (ki) se trouve le point regardé (B), puis une marque visible (20) est notée dans le diagramme à la hauteur de l'axe correspondant au moment de l'image et à la hauteur de l'autre axe correspondant à la catégorie (ki).

11. Méthode selon la revendication 9 ou 10, **caractérisée en ce qu'**en vue d'une représentation compressée de la durée de passage au point regardé (B) dans certaines catégories (ki) d'une séquence de vision sur une image fixe,
- on détermine pour chaque catégorie (ki) dans quelles images le point regardé (B) se trouve dans une catégorie (ki),
- la durée de passage au point regardé (B) dans cette catégorie (ki) est déterminée par addition de la durée de ces images, et
- une image est créée qui correspond à l'image fixe dans laquelle est disposé, au niveau de chaque catégorie (ki), un élément graphique (30) comprenant au moins un paramètre proportionnel à la durée de passage.

12. Méthode selon la revendication 9, 10 ou 11, **caractérisée en ce qu'**en vue de la représentation compressée de la succession des séquences de vision, une représentation en perspective ou sans distorsion de la scène regardée par la personne testée est créée et une flèche est associée à chaque point regardé (B).

13. Méthode selon l'une des revendications 1 à 12, **caractérisée en ce que** la situation d'examen est enregistrée et cet enregistrement est présenté à chaque personne testée, les séquences de vision de la personne étant enregistrées et évaluées par un système de détection du regard,
- le champ de vision de la personne testée étant enregistré par une première caméra (1) dirigée vers l'avant et fixée de façon rigide à la tête de la personne testée,
- les coordonnées (xb,yb) du point regardé (B), c'est-à-dire du point fixé par la personne testée, étant déterminées pour chaque image de la vidéo du champ de vision dans la vidéo du champ de vision.
